(19) Europäisches Patentamt
European Patent Office
Office européen des brevets

(11) **EP 2 534 123 B1**

(12) **FASCICULE DE BREVET EUROPEEN**

(45) Date de publication et mention de la délivrance du brevet:
**29.07.2015 Bulletin 2015/31**

(21) Numéro de dépôt: **11708901.1**

(22) Date de dépôt: **01.02.2011**

(51) Int Cl.:
*C07C 51/00* [(2006.01)]    *C07C 59/08* [(2006.01)]

(86) Numéro de dépôt international:
**PCT/FR2011/000062**

(87) Numéro de publication internationale:
**WO 2011/098683 (18.08.2011 Gazette 2011/33)**

(54) **PROCÉDÉ DE TRANSFORMATION DE BIOMASSE LIGNOCELLULOSIQUE OU DE CELLULOSE PAR DES ACIDES SOLIDES DE LEWIS A BASE DE TUNGSTENE**

VERFAHREN ZUR VERARBEITUNG EINER LIGNOZELLULOSE- ODER ZELLULOSEBIOMASSE DURCH FESTE LEWIS-SÄUREN AUF WOLFRAMBASIS

METHOD FOR PROCESSING A LIGNOCELLULOSE OR CELLULOSE BIOMASS BY MEANS OF SOLID TUNGSTEN-BASED LEWIS ACIDS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **11.02.2010 FR 1000573**

(43) Date de publication de la demande:
**19.12.2012 Bulletin 2012/51**

(73) Titulaires:
• **IFP Energies nouvelles**
  **92852 Rueil-Malmaison Cedex (FR)**
• **CNRS**
  **75794 Paris Cedex 16 (FR)**
• **Université Claude Bernard Lyon 1**
  **69622 Villeurbanne, Cedex (FR)**

(72) Inventeurs:
• **CHAMBON, Flora**
  **F-69500 Bron (FR)**
• **ESSAYEN, Nadine**
  **F-38540 Saint Just Chaleyssin (FR)**
• **RATABOUL, Franck**
  **F-69008 Lyon (FR)**
• **PINEL, Catherine**
  **F-69007 Lyon (FR)**
• **CABIAC, Amandine**
  **F-69007 Lyon (FR)**
• **GUILLON, Emmanuelle**
  **F-69390 Vernaison (FR)**

(56) Documents cités:
**EP-A1- 2 100 871**

• **PATENT ABSTRACTS OF JAPAN vol. 2010, 2010 & JP 2009, 263241, A, (NIPPON SHOKUBAI CO LTD), 12 novembre 2009 (2009-11-12)**

## Description

L'invention concerne un procédé de transformation de biomasse lignocellulosique ou de cellulose mettant en oeuvre des catalyseurs hétérogènes à base de tungstène dispersé sur un support à base d'oxyde(s), de préférence à base d'oxyde(s) d'aluminium et/ou de zirconium et/ou de titane et/ou de niobium. L'utilisation de ces catalyseurs permet d'obtenir directement de l'acide lactique à haute sélectivité tout en limitant la production d'oligosaccharides et de polymères solubles.

## Art antérieur

Depuis quelques d'années, il existe un vif regain d'intérêt pour l'incorporation de produits d'origine renouvelable au sein des filières carburant et chimie, en complément ou en substitution des produits d'origine fossile. Une voie possible est la conversion de la cellulose, contenue dans la biomasse lignocellulosique, en produits ou intermédiaires chimiques, comme l'acide lactique.

Le terme biomasse lignocellulosique (BLC) ou lignocellulose englobe plusieurs produits présents en quantités variables selon son origine : la cellulose, l'hémicellulose et la lignine. L'hémicellulose et la cellulose constituent la partie carbohydrate de la lignocellulose. Ce sont des polymères de sucres (pentoses et hexoses). La lignine est une macromolécule riche en motifs phénoliques. Par biomasse lignocellulosique, on entend par exemple les produits issus de l'exploitation forestière et les sous-produits issus de l'agriculture comme la paille ainsi que certains végétaux dédiés à haut rendement agricole.

La production de produits chimiques à partir de biomasse lignocellulosique permet à la fois de réduire la dépendance énergétique vis-à-vis du pétrole et de préserver l'environnement à travers la diminution des émissions de gaz à effet de serre sans utiliser de ressources destinées aux usages alimentaires.

La transformation directe de biomasse lignocellulosique ou de cellulose en produits ou intermédiaires chimiques, comme l'acide lactique, est une voie particulièrement intéressante. Par transformation directe, on entend la transformation cellulose - acide lactique sans passer par l'intermédiaire glucose.

L'acide lactique est un acide carboxylique, sa formule chimique est $C_3H_6O_3$ et sa structure se reflète dans son nom systématique, l'acide 2-hydroxy-propanoïque. Comme il possède un carbone asymétrique, il existe deux énantiomères de l'acide lactique. Les applications de l'acide lactique sont principalement celles de son polymère PLA (poly lactic acid) comme conservateur alimentaire mais aussi comme polymères biodégradables, pesticides et herbicides.

La production de l'acide lactique peut se faire par voie chimique ou par voie biologique. Les voies chimiques de production de l'acide lactique connues de l'homme du métier se font via la transformation d'intermédiaires pétrochimiques telles que l'hydrolyse du lactonitrile ou l'hydratation de l'acide propionique. L'acide lactique peut également être produit par fermentation de polysaccharides, qui peuvent être issus de la biomasse, par exemple issus de céréales comme le blé ou le maïs. La demande de brevet EP 1953234 concerne un procédé de production d'acide lactique par fermentation d'un extrait de canne à sucre, au moyen de micro-organisme appartenant aux genres Bacillus ou Sporolactobacillus.

La valorisation de la biomasse lignocellulosique ou de la cellulose contenue dans la biomasse en catalyse hétérogène est décrite dans la littérature. Par exemple, l'hydrolyse de la cellulose en glucose ou sorbitol en milieu aqueux sur catalyseurs métalliques hétérogènes est décrite dans la demande de brevet EP 2011569. Rinaldi et al. décrivent la dépolymérisation de la cellulose en milieu liquide ionique en présence de catalyseurs acides de Brønsted (Angew. Chem. Int. Ed., 2008, 47, 8047-8050). Zeng et al. décrivent la conversion de glucose en acide lactique, 5-(hydroxyméthyle)furfural et acide lévulinique en présence de catalyseurs basiques de types oxydes mixtes Al/Zr (Catal. Lett. (2009) 133 : 221-226).

Aussi, l'obtention d'acide lactique par traitement de la cellulose/lignocellulose en conditions hydrothermales en présence de catalyseurs homogènes basiques est connue. Par catalyse homogène basique, Fangmin Jin et Heiji Einomoto (J. Mater. Sci. (2008) 43 : 2463-2471) reportent un rendement de 27% en acide lactique en présence de Ca(OH)$_2$ à une température de 300°C en moins de 5 min de réaction. Kong et al. (J. Chem. Technol. Biotechnol. 83 : 383-388 (2008)) décrivent un procédé hydrothermal de production d'acide lactique à partir de biomasse en milieu eau subcritique en présence de cations de métaux de transition Zn(II), Ni(II), Co(II) and Cr(III)). WO 03/035582 décrit l'hydrogénolyse du sorbitol à 200°C en utilisant des catalyseurs (Ni, Re)/C qui conduit à des rendements en acide lactique de 5% et de 30% en diols (éthylène glycol et propylène glycol). Shimizu et al. (Green Chem., 2009, 11, 1627-1632) ont montré le rôle primordial de l'acidité de Brønsted sur l'hydrolyse de la cellulose en glucose. Zhang et al. (Angew. Chem. Int. Ed. 2008, 47, 8510-8513) ont étudié la transformation de la cellulose en éthylène glycol et propylène glycol sur des catalyseurs carbures de tungstène/charbon activé avec du nickel comme promoteur (T = 245°C, p = 6 MPa, H$_2$, eau).

EP2100871 décrit un procédé de préparation d'acide lactique à partir de cellulose en présence d'hydrogène, d'hydroxyde de sodium, d'eau et d'un catalyseur comprenant un métal de transition pouvant appartenir au groupe VIB. JP20092632241 décrit un procédé de préparation d'acide lactique à partir de matériaux cellulosique en présence d'eau (ou en solution alcoolique) et d'un catalyseur comprenant un oxyde de terre rares

Il n'existe pas de procédé qui permette une transformation directe, c'est à dire sans passer par l'intermédiaire glucose, de la cellulose ou plus largement de la biomasse lignocellulosique en acide lactique au moyen de catalyseurs hétérogènes. La demanderesse a découvert un procédé de transformation directe de la cellulose, présente dans la biomasse lignocellulosique, en acide lactique, mettant en oeuvre des catalyseurs hétérogènes à base de tungstène dispersé sur un support d'oxyde(s). De plus, ce procédé permet d'obtenir un rendement élevé en acide lactique.

**Objet de l'invention**

L'invention consiste en un procédé de transformation de la biomasse lignocellulosique ou de cellulose en l'acide lactique en présence d'eau, mettant en oeuvre des catalyseurs hétérogènes à base de tungstène dispersé sur un support d'oxyde(s). Le procédé permet d'obtenir des conversions élevées du réactif et des sélectivités importantes, en particulier des rendements élevés en acide lactique, tout en limitant la formation d'oligosaccharides ou de polymères hydrosolubles. Ces conversions et sélectivités ne sont obtenues qu'en conditions hydrothermales (présence d'eau) et qu'en présence de catalyseurs à base de tungstène présentant des propriétés acides de type Lewis. En effet, les catalyseurs solides ayant majoritairement une acidité de Brønsted favorisent la production d'oligosaccharides solubles et/ou polymères solubles, représentant une moindre sélectivité en intermédiaires chimiques désirés.

**Description détaillée de l'invention**

L'invention concerne un procédé de transformation de biomasse lignocellulosique ou de cellulose en acide lactique comprenant la mise en contact de la biomasse lignocellulosique ou de la cellulose en présence d'eau avec un catalyseur hétérogène à base de tungstène dispersé sur un support à base d'oxyde(s), de préférence un support à base d'oxyde(s) d'aluminium et/ou de zirconium et/ou de titane et/ou de niobium, ledit catalyseur présentant des sites acides de type Lewis. La teneur en sites acides de type Lewis du catalyseur est de préférence supérieure à 50 %. L'utilisation de ces catalyseurs permet d'obtenir directement de l'acide lactique en haute sélectivité tout en limitant la production d'oligosaccharides et polymères solubles.

**La charge**

La biomasse lignocellulosique est essentiellement constituée de trois constituants naturels présents en quantités variables selon son origine : la cellulose, l'hémicellulose et la lignine.

La cellulose ($C_6H_{10}O_5)_n$ représente la majeure partie (50-60%) de la composition de la biomasse lignocellulosique. La cellulose est un homopolymère linéaire semi-cristallin du glucose relié par des liaisons β. La cellulose est insoluble dans l'eau à température et pression ambiantes.

L'hémicellulose est le deuxième carbohydrate en quantité après la cellulose et constitue 20 à 40% en poids de la biomasse lignocellulosique. Contrairement à la cellulose, ce polymère est constitué en majorité de monomères de pentoses (cycles à cinq atomes) et hexoses (cycles à 6 atomes). L'hémicellulose est un hétéropolymère amorphe avec un degré de polymérisation inférieur à celui de la cellulose (30-100), et qui est généralement soluble dans l'eau.

La lignine est une macromolécule amorphe présente dans les composés lignocellulosiques dans des proportions variables selon l'origine du matériau (paille ~ 15%, bois : 20-26%). Sa fonction est le renforcement mécanique, l'hydrophobisation et le soutien des végétaux. Cette macromolécule riche en motifs phénoliques peut être décrite comme résultant de la combinaison de trois unités monomères de type propyl-méthoxy-phénols. Sa masse molaire varie de 5000 g/mol à 10000 g/mol pour les bois durs et atteint 20000 g/mol pour les bois tendres.

La matière première lignocellulosique peut être constituée de bois ou de déchets végétaux. D'autres exemples non limitatifs de matière biomasse lignocellulosique sont les résidus d'exploitation agricole (paille, herbes, tiges, noyaux, coquilles...), les résidus d'exploitation forestière (produits de première éclaircie, écorces, sciures, copeaux, chutes...), les produits d'exploitation forestière, les cultures dédiées (taillis à courte rotation), les résidus de l'industrie agro-alimentaire (résidu de l'industrie du cotton, bambou, sisal, banane, maïs, panicum virgatum, alfalfa, noix de coco, bagasse...), les déchets organiques ménagers, les déchets des installations de transformation du bois, les bois usagés de construction, du papier, recyclé ou non.

La charge utilisée dans le procédé selon l'invention est de la biomasse lignocellulosique ou de la cellulose. La cellulose utilisée peut être cristalline ou amorphe.

La charge biomasse lignocellulosique peut être utilisée sous sa forme brute, c'est à dire dans son intégralité de ces trois constituants cellulose, hémicellulose et lignine. La biomasse brute se présente généralement sous forme de résidus fibreux ou poudre. En général, elle est broyée pour permettre le transport de celle-ci (déchiquetage).

La charge biomasse lignocellulosique peut aussi être utilisée sous sa forme prétraitée, c'est à dire sous une forme contenant au moins une partie cellulosique après extraction de la lignine et/ou de l'hémicellulose.

La biomasse subit de préférence un prétraitement afin d'augmenter la réactivité et l'accessibilité de la cellulose au sein de la biomasse avant sa transformation. Ces prétraitements sont de nature mécanique, thermochimique, thermo-mécanico-chimique et/ou biochimique et provoquent la decristallinisation de la cellulose, la solubilisation de l'hémicellulose et/ou de la lignine ou l'hydrolyse partielle de l'hémicellulose suivant le traitement.

Les traitements mécaniques vont au delà du simple déchiquetage car ils modifient de la structure chimique des constituants. Ils améliorent l'accessibilité et la réactivité de la cellulose par sa decristallinisation et par l'augmentation de la surface d'échange. Les traitements mécaniques incluent la réduction de la taille des fibres ou particules élémentaires, par exemple par mise en copeaux de la biomasse à l'aide d'une coupeuse, par broyage de la biomasse (ajustement de la granulométrie), déstructuration des copeaux sur presse ou défibrage par abrasion des copeaux, après préchauffage. Les traitements mécaniques peuvent être opérés en mode décentralisé près de la production de la biomasse ou en mode centralisé alimentant directement la transformation.

Les traitements thermochimiques incluent la cuisson de la biomasse à haute température (150-170°C) en milieu acide dilué (principalement acide sulfurique, mais aussi acide phosphorique, acide acétique ou acide formique), en milieu alcalin (soude, sulfites, chaux...) ou en milieu oxydant (oxydation humide à l'air ou à l'oxygène ; peroxyde en milieu alcalin ; acide peracétique). Les autres traitements thermochimiques incluent des traitements aux solvants (éthanol à chaud) ou la torréfaction qui peut se définir comme une pyrolyse à température modérée et temps de séjour contrôlé car elle s'accompagne d'une destruction partielle de la matière lignocellulosique. Les technologies connues pour la torréfaction sont par exemple le four tournant, le lit mobile, le lit fluidisé, la vis sans fin chauffée, le contact avec de billes métalliques apportant la chaleur. Ces technologies peuvent éventuellement utiliser un gaz circulant à co ou contre courant comme de l'azote ou tout autre gaz inerte dans les conditions de la réaction.

Les traitements thermo-mécanico-chimiques incluent les traitements à la vapeur (explosion à la vapeur appelés encore hydrolyse flash ou "steam-explosion"), le traitement AFEX (ammonia fiber explosion) à l'ammoniaque ou l'extrusion bi-vis avec des réactifs chimiques divers.

Le prétraitement permet de préparer la biomasse lignocellulosique en séparant la partie carbohydrate de la lignine et ajustant la taille des particules de biomasse à traiter. La taille des particules de biomasse après le prétraitement est généralement inférieure à 5 mm, de préférence inférieure à 500 microns.

**Le catalyseur**

Les catalyseurs utilisés pour la transformation de la biomasse lignocellulosique ou de la cellulose sont à base d'oxyde de tungstène dispersé à la surface d'un support d'oxyde(s), de préférence un support d'oxyde(s) d'aluminium et/ou de zirconium et/ou de titane et/ou de niobium, lesdits catalyseurs présentant des sites acides de type Lewis.

D'une manière générale, l'acidité d'un catalyseur est la résultante de deux types d'acidité combinés : une acidité de Lewis, caractérisée par la présence d'une lacune électronique sur un atome, et une acidité de Bronsted, caractérisée par une aptitude à céder un proton. La nature des sites acides peut se caractériser par adsorption de pyridine suivie par spectroscopie IR conformément à la méthode décrite dans *[M. Guisnet, P. Ayrault, C. Coutanceau, M.F. Alvarez, J. Datka, J. Chem. Soc., Faraday Trans. 93, 1661 (1997)]*.

Les solides selon l'invention sont caractérisés par des propriétés acides superficielles majoritairement de type Lewis, de préférence supérieur à 50 %. Les sites acides de type Lewis sont associés à la présence d'espèces tungstène coordinativement insaturées mais aussi aux espèces caractéristiques du support : $Al^{3+}$, $Zr^{4+}$, $Ti^{4+}$, $Nb^{5+}$. Il est connu que la coordination des espèces tungstène de surface (tétraédrique/octaédrique) dépendent de leur dispersion, de la teneur en W, de la nature des précurseurs et des traitements thermiques.

Les catalyseurs de type ZrW, associés ou non à une phase métallique, sont décrits pour être actifs dans de nombreuses applications telles que l'hydroisomérisation des paraffines (US 6124232) ou la dimérisation d'oléfines (US 5453556). La zircone tungstée est préparée de façon usuelle par imprégnation ou coprécipitation : les oxydes de tungstène supportés sur zircone ont été décrits pour la première fois par Hino et Arata (J. Chem. Soc., Chem. Commun., 1148 (1979)). Ce solide est obtenu par imprégnation de zircone par le metatungstate d'ammonium, suivi d'une décomposition sous air à 800-850°C. Le brevet US 5510309 divulgue un solide obtenu par coprécipitation de metatungstate d'ammonium et de $ZrOCl_2$, suivie d'une calcination à une température supérieure à 700°C.

Bien que les catalyseurs tungstène/Zr et/ou Al et/ou Ti et/ou Nb soient connus, ils n'ont jamais été appliqués dans la conversion de matières lignocellulosiques ou de cellulose en présence d'eau et plus particulièrement pour produire sélectivement des intermédiaires chimiques importants comme l'acide lactique.

Les catalyseurs utilisés dans le procédé selon la présente invention peuvent être synthétisés par échange ionique, par imprégnation ou par coprécipitation suivi d'un traitement thermique. Les solides obtenus présentent les avantages d'être mésoporeux et stables, thermiquement et en conditions hydrothermales.

Les catalyseurs utilisés dans la présente invention peuvent être sous forme de poudre, d'extrudés, de billes ou de pastilles.

Les supports d'oxyde(s) sont de préférence choisis parmi les oxydes/hydroxydes d'aluminium et/ou de zirconium et/ou de titane et/ou de niobium.

La teneur en tungstène est comprise entre 2 à 30% en poids, de préférence entre 10 et 20% poids ou encore de préférence entre 2 à 20 % en poids, les pourcentages étant exprimés en % poids de métal par rapport à la masse totale du catalyseur.

Les précurseurs de tungstène sont choisis parmi l'acide tungstique, l'acide peroxotungstique, le méta tungstate d'ammonium, ou les isopolyanions ou hétéropolyanions à base de tungstène. Le méta tungstate d'ammonium est le précurseur usuel. L'utilisation d'acide tungstique en solution dans du peroxyde d'hydrogène est préférée car on favorise par cette méthode la formation d'espèces tungstènes monomériques en solution, espèces échangeables à pH acide avec les supports à base de Zr, Ti, Al et/ou Nb selon l'invention WO 2004/004893.

La méthode de préparation préférée consiste en un échange anionique entre une solution d'acide tungstique dans du peroxyde d'hydrogène et l'hydroxyde de zirconium et/ou de titane et/ou d'aluminium et/ou de niobium, suivie d'une calcination selon US 2006/0091045.

## Procédé de transformation

Le procédé de transformation de la biomasse lignocellulosique ou de la cellulose selon l'invention comprend la réaction dans un milieu contenant de l'eau en présence de la composition catalytique conforme à l'invention.

Par milieu contenant de l'eau on désigne les milieux liquides conventionnels (comme par exemple l'éthanol ou l'eau) et les milieux non conventionnels comme les liquides ioniques ou les milieux supercritiques de densité type liquide.

La teneur massique en eau dans le milieu est généralement supérieure à 1%. Le milieu peut aussi consister entièrement d'eau. De préférence, le milieu est de l'eau.

Ce procédé peut être effectué en présence d'un gaz choisi parmi l'air, un gaz neutre ($N_2$, He, Ar...) ou un gaz réducteur comme l'hydrogène. De préférence, l'hydrogène est utilisé car on observe une meilleure conversion de la cellulose et un meilleur rendement en acide lactique.

Le procédé est opéré à des températures comprises entre 160°C et 250°C, de préférence entre 175 et 250°C, et à une pression comprise entre 0.5 et 20 MPa, de préférence entre 2 et 10 MPa.

Généralement la réaction peut être opérée selon différents modes de réalisation. Ainsi, la réaction peut être mise en oeuvre en discontinu ou en continu, par exemple en lit fixe. On peut opérer en réacteur fermé ou semi-ouvert.

Le catalyseur est introduit dans le procédé à raison d'une quantité correspondant à un rapport massique biomasse/catalyseur compris entre 1 et 1000, de préférence entre 1 et 500, de préférence entre 1 et 100, de préférence entre 1 et 50 et encore préférentiellement entre 1 et 25.

La biomasse est introduite dans le procédé à raison d'une quantité correspondant à un rapport massique (milieu contenant de l'eau)/biomasse compris entre 1 et 1000, de préférence entre 1 et 500 et encore préférentiellement entre 5 et 100. Le taux de dilution de la biomasse est entre 1:1 et 1:1000, de préférence entre 1:1 et 1:500 et encore préférentiellement entre 1:5 et 1:100.

Si l'on choisit un procédé en continu, la vitesse massique horaire (débit de charge massique/masse de catalyseur) est entre 0.01 et 5 $h^{-1}$, de préférence entre 0.02 et 2 $h^{-1}$.

## Les produits obtenus et leur mode d'analyse

Après la réaction, le milieu réactionnel est prélevé et centrifugé. Le liquide réactionnel est ensuite analysé par chromatographie liquide sous haute pression (HPLC) en utilisant la réfractométrie pour déterminer la teneur en produits de conversion de la solution aqueuse.

Les produits de la réaction sont solubles dans l'eau. Ils sont constitués de monosaccharides et de leurs dérivés, d'oligosaccharides, mais aussi de polymères solubles formés par combinaisons successives des dérivés des monosaccharides.

Par monosaccharides, on désigne les sucres simples (hexoses, pentoses) produits par dépolymérisation complète de la cellulose et/ou hémicellulose, en particulier, le glucose, le mannose, le xylose, le fructose....

Par dérivés des monosaccharides on désigne les produits pouvant être obtenus par déshydratation, isomérisation, réduction ou oxydation :

- des sucres alcools, des alcools et polyols : en particulier le sorbitol, le xylitol, le glycérol, l'éthylène glycol, le propylène glycol, l'éthanol, le Me-butane diols...
- des cétones, des hexane-diones : la 2,5-hexanedione...
- des acides carboxyliques et leurs esters, des lactones : l'acide formique, acide lévulinique, lévulinates d'alkyles, acide lactique, lactique d'alkyles, acide glutarique, glutarates d'alkyles, l'acide 3-hydroxypropanoique, la 3-hydroxybutyrolactone, la γ-butyrolactone,
- des éthers cycliques : le THF, le Me-THF, le furane dicarboxylique acide, le 5-(hydroxyméthyle)furfural.

Par oligosaccharides, on désigne un carbohydrate ayant pour composition $(C_6H_{10}O_5)_n$ où n est supérieur à 1 obtenus par hydrolyse partielle de la cellulose, ou de l'hémicellulose, ou de l'amidon.

Par polymères solubles, on désigne tous les produits issus de la condensation entre monosaccharides, oligosaccharides et/ou dérivés des monosaccharides.

La quantité des produits de réaction solubles dans l'eau (monosaccharides et dérivés, oligosaccharides, polymères solubles) est déterminée par l'analyse COT (Carbone Organique Total) qui consiste en la mesure du carbone en solution. La quantité des monosaccharides et leurs dérivés est déterminée par analyses HPLC.

La conversion (équivalent au % de solubilisation) de la biomasse ou de la cellulose est calculée suivant l'équation suivante :

$$C = 100 * C_{solubilisé} / C_{initial}$$

dans laquelle $C_{solubilisé}$ représente la quantité de carbone solubilisé analysée par COT (mg) et $C_{initial}$ la quantité de carbone en début de réaction contenue dans la biomasse ou cellulose solide.

Les rendements molaires en dérivés du glucose sont calculés au moyen de l'analyse HPLC. Chaque composé est corrigé du nombre d'atome de carbone contenu dans l'unité glucose.

Les rendements molaires en un dérivé i sont calculés comme suit :

$$Rdti = 100* (nC_{Pi}/6)*( P_i/Glu_0)$$

où $nC_{pi}$ représente le nombre d'atome de carbones du dérivé i, Pi le nombre de moles du produit $P_i$ et $Glu_0$ le nombre de moles d'unités glucose contenues dans la biomasse ou la cellulose en début de réaction.

La formation d'oligosaccharides et de polymères solubles correspondent à une perte de carbone. Cette perte de carbone est déduite des analyses COT et HPLC. Le rendement en oligosaccharides et polymères solubles est calculé selon l'équation suivante :

$$Rdt_{olig} = C - \Sigma rdt_i$$

où C représente la conversion de la cellulose et $\Sigma rdt_i$ la somme des rendements molaires de tous les monosaccharides et leurs dérivés analysés par HPLC.

**Exemples**

Préparation de deux catalyseurs selon l'invention :

Exemple 1 : Préparation d'une zircone tungstée

De l'hydroxyde de zirconium ($Zr(OH)_4$) est préparé dans une première étape. Il est préparé par précipitation à un pH constant de 9 à partir d'une solution 0.4 M de chlorure de zirconyle ($ZrOCl_2$, $8H_2O$) et d'une solution d'ammoniaque 1.8 M. La suspension obtenue est agitée pendant 20 minutes. Le précipité est séparé par centrifugation et lavé plusieurs fois à l'eau dé ionisée afin d'éliminer les ions chlorures. Le solide obtenu est ensuite séché à 110°C pendant 24h.

La zircone tungstée a été synthétisée conformément à l'enseignement du brevet US2006/0091045. Cet hydroxyde de zirconium, séché, (10g) est ensuite soumis à un échange ionique pendant 15 min en utilisant une solution d'acide tungstique 0.25 M dans le peroxyde d'hydrogène à 30% (150 mL). Après cet échange, le solide est filtré puis séché à 80°C pendant 24h.

Ensuite, le solide obtenu est calciné sous débit d'air sec à la température de 700°C pendant 3h.

A l'issu de ces traitements, la zircone tungstée ainsi obtenue contient en poids 11.7% de tungstène. La nature des sites acides de ce catalyseur a été caractérisée par adsorption de pyridine suivie par spectroscopie IR conformément la méthode décrite dans [M. Guisnet, P. Ayrault, C. Coutanceau, M.F. Alvarez, J. Datka, J. Chem. Soc., Faraday Trans. 93, 1661 (1997)]. Plus de 65% des sites acides de cette formulation catalytique à base de tungstène sont des sites acides de type Lewis.

<u>Exemple 2 : Préparation d'une alumine tungstée (18 % poids de tungstène)</u>

La matière première utilisée est l'hydroxyde d'aluminium (Boehmite). Cet hydroxyde d'aluminium (10g) est soumis à un échange anionique à l'aide d'acide tungstique en solution (0.25 M) dans le peroxyde d'hydrogène à 30% (150 mL). L'échange dure 15 minutes à température ambiante. Après cet échange, le solide est filtré puis séché à 80°C pendant 24h.

Ensuite, le solide obtenu est calciné sous débit d'air sec à la température de 700°C pendant 3h.

A l'issu de ces traitements, l'alumine tungstée ainsi obtenue contient en poids 18% de tungstène.

La nature des sites acides de ce catalyseur a été caractérisée par la méthode décrite dans l'exemple 1. Plus de 90% des sites acides de cette formulation catalytique à base de tungstène sont des sites acides de type Lewis.

<u>Préparation d'un catalyseur non-conforme à l'invention (acide de Brønsted) :</u>

<u>Exemple 3 : Préparation d'un sel acide de césium de l'acide 12-tungstophosphorique $H_3PW_{12}O_{40}$</u>

La matière première utilisée est l'acide 12-tungstophosphorique hydraté $H_3PW_{12}O_{40}.21H_2O$ et du chlorure de césium. Le sel acide de césium $Cs_2HPW_{12}O_{40}$ est préparé par précipitation par ajout d'une solution aqueuse de chlorure de césium (5M) à une solution de $H_3PW_{12}O_{40}$ dans de l'eau (0.5M) à raison de quantités correspondant à un rapport molaire $CsCl/H_3PW_{12}O_{40}$ de 2. La suspension aqueuse est laissée sous agitation pendant 12h à température ambiante. Le solide précipité est ensuite séparé par centrifugation et lavé trois fois à l'eau dé ionisée entre chaque centrifugation. Le solide obtenu est séché par lyophilisation.

La nature des sites acides de ce catalyseur a été caractérisée par la méthode décrite dans l'exemple 1. 100% des sites acides de cette formulation catalytique à base de tungstène sont des sites acides de type Brønsted.

<u>Exemple 4 : Transformation de la cellulose mettant en oeuvre les catalyseurs selon l'invention et des catalyseurs non-conformes à l'invention</u>

Cet exemple concerne la conversion de la cellulose à partir de différents catalyseurs pour la production d'acide lactique.

On introduit dans un autoclave de 100 mL 65 ml d'eau, 1.6g de cellulose Avicel® (70% cristallinité) et 0.68 g de catalyseur des exemples 1 à 3. Le rapport massique cellulose/catalyseur est de 2.35. On chauffe à 190°C et on injecte 5 MPa $H_2$. La pression totale atteint alors 6 MPa. Après 24h de réaction, le milieu réactionnel est prélevé et centrifugé. Le liquide réactionnel est ensuite analysé par chromatographie liquide sous haute pression (HPLC) en utilisant la réfractométrie pour déterminer la teneur en produits de conversion de la solution aqueuse selon les équations décrites ci-dessus.

La réaction de transformation a également été effectuée avec les supports d'oxydes non tungstée $Al_2O_3$ ou $ZrO_2$.

Les résultats obtenus sont référencés dans le tableau 1.

Tableau 1 : Conversion de cellulose et rendements en acide lactique et oligosaccharides et polymères solubles utilisant différents catalyseurs.

| catalyseur | conversion cellulose (%) | rendement molaire en acide lactique (%) | rendements molaires en oligosaccharides et polymères solubles (%) |
|---|---|---|---|
| $Cs_2HPW_{12}O_{40}$ (exemple 3, non conforme) | 32 | 5 | 15 |
| $Al_2O_3$ (non conforme) | 35 | 3 | 7 |
| AlW (18% pds W) (exemple 2) | 55 | 28 | 7 |
| AlW (5% pds W) (exemple 5) | 35 | 19 | 0 |
| $ZrO_2$ (non conforme) | 43 | 2 | 19 |
| ZrW (exemple 1) | 67 | 14 | 0 |

De manière générale, on trouve que pour le catalyseur $Cs_2HPW_{12}O_{40}$, la quantité d'acide lactique formé représente 5% en mole de la quantité de cellulose de départ, avec 15% en mole d'oligosaccharides et polymères solubles. La conversion est de 32%.

On trouve ainsi que pour le catalyseur AlW à 18 %pds W, la quantité d'acide lactique formé représente 28% en mole de la quantité de cellulose de départ, avec 7% en mole d'oligosaccharides et polymères solubles. La conversion est de 55%.

On trouve que pour le catalyseur ZrW, la quantité d'acide lactique formé représente 14% en mole de la quantité de cellulose de départ, sans formation d'oligosaccharides et polymères solubles. La conversion est de 67%.

L'influence du composé tungstène dans la production d'acide lactique se montre dans la comparaison des essais des catalyseurs tungstées aux supports d'oxydes sans tungstène ($Al_2O_3$ et $ZrO_2$). On observe un rendement molaire en acide lactique 9 fois (7 fois) plus important en présence de tungstène dans le cas de l'aluminium (zirconium). Ceci est du à la présence de sites acides de type Lewis sur ces catalyseurs.

Préparation d'un catalyseur selon l'invention ayant une faible teneur en tungstène

Exemple 5 : Préparation d'une alumine tungstée (5 % poids de tungstène)

La matière première utilisée est l'hydroxyde d'aluminium (Boehmite). Cet hydroxyde d'aluminium (10g) est soumis à un échange anionique à l'aide d'acide tungstique en solution (0.12 M) dans le peroxyde d'hydrogène à 30% (150 mL). L'échange dure 15 minutes à température ambiante. Après cet échange, le solide est filtré puis séché à 80°C pendant 24h.

Ensuite, le solide obtenu est calciné sous débit d'air sec à la température de 700°C pendant 3h.

A l'issu de ces traitements, l'alumine tungstée ainsi obtenue contient en poids 5% de tungstène.

La nature des sites acides de ce catalyseur a été caractérisée par la méthode décrite dans l'exemple 1. Plus de 90% des sites acides de cette formulation catalytique à base de tungstène sont des sites acides de type Lewis.

Les résultats obtenus sont référencés dans le tableau 1.

On trouve ainsi que pour le catalyseur AlW à 5% pds W, la quantité d'acide lactique formé représente 19% en mole de la quantité de cellulose de départ, sans formation d'oligosaccharides et polymères solubles. La conversion est de 35%.

En comparant ces résultats avec les résultats obtenus avec le catalyseur AlW à 18% pds W, on constate qu'une teneur plus faible de composé tungstée permet d'obtenir une conversion respectable, une bonne sélectivité en acide lactique sans formation d'oligosaccharides et polymères solubles. Il est donc possible d'utiliser un catalyseur ayant un coût moindre.

Exemple 6 : Transformation de la cellulose mettant en oeuvre le catalyseur AlW à différents rapports massiques biomasse/catalyseur

L'exemple 4 est répété avec le catalyseur AlW à 18% pds W de l'exemple 2 en utilisant un rapport massique cellulose/AlW de 23.5.

On introduit dans un autoclave de 100 mL 65 ml d'eau, 1.6g de cellulose Avicel® (70% cristallinité) et 0.068 g de catalyseur AlW. On chauffe à 190°C et on injecte 5 MPa $H_2$. La pression totale atteint alors 6 MPa. Après 24h de réaction, le milieu réactionnel est prélevé et centrifugé. Le liquide réactionnel est ensuite analysé par chromatographie liquide sous haute pression (HPLC) en utilisant la réfractométrie pour déterminer la teneur en produits de conversion de la solution aqueuse selon les équations décrites ci-dessus.

Les résultats obtenus sont référencés dans le tableau 2.

Tableau 2 : Conversion de cellulose et rendements en acide lactique et oligosaccharides et polymères solubles utilisant le catalyseur AlW à 18% pds W à différents rapports massiques biomasse/catalyseur.

|  | catalyseur | rapport massique cellulose/catalyseur | conversion cellulose (%) | rendement molaire en acide lactique (%) | rendements molaires en oligosaccharides et polymères solubles (%) |
|---|---|---|---|---|---|
| ex. 4 | AlW (ex. 2) | 2.35 | 55 | 28 | 7 |
| ex. 6 | AlW (ex. 2) | 23.5 | 40 | 17 | 1 |

On trouve ainsi que pour un rapport cellulose/AlW de 23.5, la quantité d'acide lactique formé représente 17% en mole de la quantité de cellulose de départ, avec uniquement 1% en mole d'oligosaccharides et polymères solubles.

La conversion est de 40%.

Un rapport cellulose/catalyseur plus élevé permet malgré la plus faible quantité de catalyseur d'obtenir une conversion respectable, une bonne sélectivité en acide lactique avec très peu de formation d'oligosaccharides et polymères solubles.

Ainsi, ces exemples démontrent l'obtention d'acide lactique à haut rendement par transformation directe de cellulose via des catalyseurs hétérogènes à base de tungstène tout en limitant la formation d'oligosaccharides et de polymères solubles.

## Revendications

1. Procédé de transformation de biomasse lignocellulosique ou de cellulose en acide lactique **caractérisé en ce qu'**il comprend la mise en contact de la biomasse lignocellulosique ou de la cellulose en présence d'eau avec un catalyseur hétérogène à base de tungstène dispersé sur un support à base d'oxyde(s), ledit catalyseur présentant des sites acides de type Lewis.

2. Procédé selon la revendication 1 dans lequel ledit support à base d'oxyde(s) est choisi parmi le groupe formé par les oxyde(s) d'aluminium et/ou de zirconium et/ou de titane et/ou de niobium.

3. Procédé selon les revendications 1 et 2 dans lequel la teneur en sites acides de Lewis du catalyseur est supérieure à 50 %.

4. Procédé selon l'une des revendications précédentes dans lequel la teneur en tungstène du catalyseur est comprise entre 2 et 30 % en poids, de préférence entre 10 à 20 % en poids ou encore de préférence entre 2 à 20 % en poids, les pourcentages étant exprimés en % poids de métal par rapport à la masse totale du catalyseur.

5. Procédé selon l'une des revendications précédentes dans lequel la transformation est mise en oeuvre dans un milieu contenant de l'eau, ledit milieu étant choisi parmi le groupe formé par un milieu liquide, de préférence l'éthanol ou l'eau, un liquide ionique et un milieu supercritique de densité type liquide.

6. Procédé selon la revendication 5 dans lequel la teneur massique en eau dans le milieu est supérieure à 1%.

7. Procédé selon l'une des revendications 5 et 6 dans lequel ledit milieu est de l'eau.

8. Procédé selon l'une des revendications précédentes dans lequel la transformation est effectuée à une température comprise entre 160 et 250°C, de préférence entre 175 et 250°C, et à une pression comprise entre 0.5 et 20 MPa, de préférence entre 2 et 10 MPa.

9. Procédé selon l'une des revendications précédentes dans lequel la transformation est effectuée en présence d'un gaz choisi parmi l'air, un gaz neutre ou un gaz réducteur, de préférence l'hydrogène.

10. Procédé selon l'une des revendications précédentes dans lequel le catalyseur est introduit à raison d'une quantité correspondant à un rapport massique biomasse/catalyseur compris entre 1 et 1000, de préférence entre 1 et 500, de préférence entre 1 et 100, de préférence entre 1 et 50, préférentiellement entre 1 et 25.

11. Procédé selon l'une des revendications précédentes dans lequel la biomasse ou cellulose est introduite à raison d'une quantité correspondant à un rapport massique (milieu contenant de l'eau)/ biomasse compris entre 1 et 1000, de préférence entre 1 et 500, préférentiellement entre 5 et 100.

12. Procédé selon l'une des revendications précédentes dans lequel le catalyseur hétérogène à base de tungstène est préparé par échange ionique, imprégnation ou par coprécipitation, suivi d'un traitement thermique.

13. Procédé selon la revendication 12 dans lequel le précurseur de tungstène du catalyseur est choisi parmi le groupe formé par l'acide tungstique, l'acide peroxotungstique, le méta tungstate d'ammonium ou les isopolyanions ou hétéropolyanions à base de tungstène.

14. Procédé selon l'une des revendications précédentes dans lequel le catalyseur hétérogène à base de tungstène est préparé par un échange anionique entre une solution d'acide tungstique dans du peroxyde d'hydrogène et l'hydroxyde de Zr et/ou Ti et/ou Al et/ou Nb, suivie d'une calcination.

15. Procédé selon l'une des revendications précédentes dans lequel la biomasse lignocellulosique subit avant sa transformation une étape de prétraitement mécanique, thermochimique, thermo-mécanico-chimique ou biochimique.

16. Procédé selon la revendication 15 dans lequel la taille des particules de biomasse après le prétraitement est inférieure à 5 mm, de préférence inférieure à 500 microns.

**Patentansprüche**

1. Verfahren zur Umwandlung von lignocellulosehaltiger Biomasse oder von Cellulose in Milchsäure, **dadurch gekennzeichnet, dass** es das Inkontaktbringen der lignocellulosehaltigen Biomasse oder der Cellulose in Gegenwart von Wasser mit einem heterogenen Katalysator auf der Basis von Wolfram umfasst, der auf einem Träger auf der Basis von Oxid(en) dispergiert ist, wobei der Katalysator Lewis-Säurezentren aufweist.

2. Verfahren nach Anspruch 1, wobei der Träger auf der Basis von Oxid(en) ausgewählt ist aus der Gruppe gebildet aus Aluminium- und/oder Zirconium- und/oder Titan- und/oder Nioboxid(en).

3. Verfahren nach den Ansprüchen 1 und 2, wobei der Gehalt an Lewis-Säurezentren des Katalysators größer als 50 % ist.

4. Verfahren nach einem der vorhergehenden Ansprüche, wobei der Wolframgehalt des Katalysators im Bereich zwischen 2 und 30 Gew.-%, bevorzugt zwischen 10 und 20 Gew.-% oder bevorzugt auch zwischen 2 und 20 Gew.-% liegt, wobei die Prozentsätze in Gew.-% des Metalls, bezogen auf die Gesamtmasse des Katalysators, ausgedrückt werden.

5. Verfahren nach einem der vorhergehenden Ansprüche, wobei die Umwandlung in einem Medium, das Wasser enthält, durchgeführt wird, wobei das Medium ausgewählt ist aus der Gruppe gebildet aus einem flüssigen Medium, bevorzugt Ethanol oder Wasser, einer ionischen Flüssigkeit und einem superkritischen Medium mit der Dichte einer Flüssigkeit.

6. Verfahren nach Anspruch 5, wobei der Massegehalt an Wasser in dem Medium größer als 1 % ist.

7. Verfahren nach einem der Ansprüche 5 und 6, wobei das Medium Wasser ist.

8. Verfahren nach einem der vorhergehenden Ansprüche, wobei die Umwandlung bei einer Temperatur im Bereich zwischen 160 und 250 °C, bevorzugt zwischen 175 und 250 °C, und bei einem Druck im Bereich zwischen 0,5 und 20 MPa, bevorzugt zwischen 2 und 10 MPa, durchgeführt wird.

9. Verfahren nach einem der vorhergehenden Ansprüche, wobei die Umwandlung in Gegenwart eines Gases, ausgewählt aus Luft, einem neutralen Gas oder einem Reduktionsgas, bevorzugt Wasserstoff, durchgeführt wird.

10. Verfahren nach einem der vorhergehenden Ansprüche, wobei der Katalysator in einer Menge eingeführt wird, die einem Masseverhältnis von Biomasse/Katalysator zwischen 1 und 1.000, bevorzugt zwischen 1 und 500, bevorzugt zwischen 1 und 100, bevorzugt zwischen 1 und 50, vorzugsweise zwischen 1 und 25 entspricht.

11. Verfahren nach einem der vorhergehenden Ansprüche, wobei die Biomasse oder die Cellulose in einer Menge eingeführt wird, die einem Masseverhältnis (Wasser enthaltendes Medium)/ Biomasse zwischen 1 und 1.000, bevorzugt zwischen 1 und 500, vorzugsweise zwischen 5 und 100 entspricht.

12. Verfahren nach einem der vorhergehenden Ansprüche, wobei der heterogene Katalysator auf der Basis von Wolfram durch Ionenaustausch, Imprägnierung oder durch Copräzipitation, gefolgt von einer Wärmebehandlung, hergestellt wird.

13. Verfahren nach Anspruch 12, wobei der Wolframvorläufer des Katalysators ausgewählt ist aus der Gruppe, gebildet aus Wolframsäure, Peroxowolframsäure, Ammoniummetawolframat oder den Isopolyanionen oder Heteropolyanionen auf der Basis von Wolfram.

**14.** Verfahren nach einem der vorhergehenden Ansprüche, wobei der heterogene Katalysator auf der Basis von Wolfram durch einen Anionenaustausch zwischen einer Wolframsäurelösung in Wasserstoffperoxid und dem Hydroxid von Zr und/oder Ti und/oder Al und/oder Nb, gefolgt von einer Kalzinierung, hergestellt wird.

**15.** Verfahren nach einem der vorhergehenden Ansprüche, wobei die lignocellulosehaltige Biomasse vor ihrer Umwandlung einem Schritt zur mechanischen, thermochemischen, thermomechanischen oder biochemischen Vorbehandlung unterzogen wird.

**16.** Verfahren nach Anspruch 15, wobei die Größe der Biomassepartikel nach der Vorbehandlung kleiner als 5 mm, bevorzugt kleiner als 500 Mikron ist.

**Claims**

**1.** Process for transformation of lignocellulosic biomass or cellulose into lactic acid, **characterized in that** it comprises putting the lignocellulosic biomass or cellulose in the presence of water into contact with a tungsten-based heterogeneous catalyst that is dispersed on an oxide-based substrate, with said catalyst having Lewis-type acid sites.

**2.** Process according to Claim 1, wherein said oxide-based substrate is selected from among the group that is formed by the oxide(s) of aluminum and/or zirconium and/or titanium and/or niobium.

**3.** Process according to Claims 1 and 2, wherein the content of Lewis acid sites of the catalyst is greater than 50%.

**4.** Process according to one of the preceding claims, wherein the tungsten content of the catalyst is between 2 and 30% by weight, preferably between 10 to 20% by weight, or else preferably between 2 to 20% by weight, with the percentages being expressed in % by weight of metal relative to the total mass of catalyst.

**5.** Process according to one of the preceding claims, wherein the transformation is implemented in a water-containing medium, with said medium being selected from among the group that is formed by a liquid medium, preferably ethanol or water, an ionic liquid and a supercritical medium of liquid-type density.

**6.** Process according to Claim 5, wherein the content by mass of water in the medium is greater than 1%.

**7.** Process according to one of Claims 5 and 6, wherein said medium is water.

**8.** Process according to one of the preceding claims, wherein the transformation is carried out at a temperature of between 160 and 250°C, preferably between 175 and 250°C, and at a pressure of between 0.5 and 20 MPa, preferably between 2 and 10 MPa.

**9.** Process according to one of the preceding claims, wherein the transformation is carried out in the presence of a gas that is selected from among air, a neutral gas or a reducing gas, preferably hydrogen.

**10.** Process according to one of the preceding claims, wherein the catalyst is introduced at a rate of a quantity that corresponds to a biomass/catalyst mass ratio of between 1 and 1,000, preferably between 1 and 500, preferably between 1 and 100, preferably between 1 and 50, and preferably between 1 and 25.

**11.** Process according to one of the preceding claims, wherein the biomass or cellulose is introduced at a rate of a quantity that corresponds to a (water-containing medium)/biomass mass ratio of between 1 and 1,000, preferably between 1 and 500, and preferably between 5 and 100.

**12.** Process according to one of the preceding claims, wherein the tungsten-based heterogeneous catalyst is prepared by ion exchange, impregnation or by co-precipitation, followed by a heat treatment.

**13.** Process according to Claim 12, wherein the tungsten precursor of the catalyst is selected from among the group that is formed by tungstic acid, peroxotungstic acid, ammonium metatungstate, or tungsten-based isopolyanions or heteropolyanions.

**14.** Process according to one of the preceding claims, wherein the tungsten-based heterogeneous catalyst is prepared by an anion exchange between a tungstic acid solution in hydrogen peroxide and hydroxide of Zr and/or Ti and/or Al and/or Nb, followed by calcination.

15. Process according to one of the preceding claims, wherein before its transformation, the lignocellulosic biomass undergoes a stage of mechanical, thermochemical, thermal-mechanical-chemical or biochemical pretreatment.

16. Process according to Claim 15, wherein the size of the biomass particles after pretreatment is less than 5 mm, preferably less than 500 microns.

## RÉFÉRENCES CITÉES DANS LA DESCRIPTION

**Documents brevets cités dans la description**

- EP 1953234 A
- EP 2011569 A, Rinaldi
- WO 03035582 A
- EP 2100871 A
- JP 20092632241 B
- US 6124232 A
- US 5453556 A
- US 5510309 A
- WO 2004004893 A
- US 20060091045 A

**Littérature non-brevet citée dans la description**

- *Angew. Chem. Int. Ed.,* 2008, vol. 47, 8047-8050
- *Catal. Lett.,* 2009, vol. 133, 221-226
- **FANGMIN JIN ; HEIJI EINOMOTO.** *J. Mater. Sci.,* 2008, vol. 43, 2463-2471
- **KONG et al.** *J. Chem. Technol. Biotechnol.,* 2008, vol. 83, 383-388
- **SHIMIZU et al.** *Green Chem.,* 2009, vol. 11, 1627-1632
- **ZHANG et al.** *Angew. Chem. Int. Ed.,* 2008, vol. 47, 8510-8513
- **M. GUISNET ; P. AYRAULT ; C. COUTANCEAU ; M.F. ALVAREZ ; J. DATKA.** *J. Chem. Soc., Faraday Trans.,* 1997, vol. 93, 1661
- **HINO ; ARATA.** *J. Chem. Soc., Chem. Commun.,* 1979, 1148